# EUROPEAN PATENT APPLICATION

(11) **EP 4 732 781 A1**
(43) Date of publication of application: **29.04.2026**
(21) Application number: 25210757.8
(22) Date of filing: 23.10.2025
(51) Int. Cl.: A61B 8/08, A61B 8/12, A61B 8/14, A61B 8/00

(54) **TREATMENT SUPPORT APPARATUS AND TREATMENT SUPPORT PROGRAM**

(30) Priority: 28.10.2024 JP 2024189254; 01.09.2025 JP 2025144897
(71) Applicant: FUJIFILM Corporation, Tokyo 106-8620 (JP)
(72) Inventor: ASAFUSA, Katsunori, Tokyo, 106-8620 (JP); DOKKO, Shuichi, Tokyo, 106-8620 (JP); ISHIDA, Koichi, Tokyo, 106-8620 (JP)
(74) Representative: Meissner Bolte Partnerschaft mbB

(57) **Abstract**

It is possible to easily specify a region of a treatment target portion in ultrasound volume data.

A three-dimensional model formation unit forms a three-dimensional model of a target tissue. The three-dimensional model has information indicating a region occupied by a treatment target portion in the target tissue. A volume data formation unit forms ultrasound volume data based on reception signals obtained by transmitting and receiving ultrasound to and from the target tissue with an ultrasound probe 18a. The ultrasound volume data is defined in an ultrasound data coordinate system based on a position and an orientation of the ultrasound probe. A coordinate system conversion unit defines the three-dimensional model in the ultrasound data coordinate system by performing alignment between the three-dimensional model and the ultrasound volume data. A treatment target portion specifying unit specifies a region of a treatment target portion in the ultrasound volume data based on the treatment target portion included in the three-dimensional model defined in the robot coordinate system.

## Description

### BACKGROUND OF THE INVENTION

### 1. Field of the Invention

The present specification discloses improvements in a treatment support apparatus and a treatment support program.

### 2. Description of the Related Art

In the related art, in a case in which a treatment target portion of a subject is treated using a treatment tool (for example, scissors or forceps), an ultrasound diagnostic apparatus may be used for an operator, such as a doctor, to check the treatment target portion. An ultrasound diagnostic apparatus is an apparatus that transmits ultrasound from an ultrasound probe toward the subject, receives a reflected wave from the subject in the ultrasound probe, and performs various processes such as the formation of an ultrasound tomographic image representing a cross-section in the subject, the formation of a Doppler image representing a flow velocity of a tissue component (blood or the like) in the subject, and various kinds of measurement based on a reception signal formed from the reflected wave.

In the related art, various techniques related to an ultrasound diagnostic apparatus used in treatment on a treatment target portion have been proposed.

For example, JP2013-255658A discloses an ultrasound diagnostic apparatus comprising: a region-of-interest (ROI) mark setting unit that sets a region of interest designated by an examiner on an ultrasound image; a camera that acquires a camera image by imaging a subject to which a subject AR mark is attached and an ultrasound probe to which an ultrasound probe AR mark is attached; a non-subject coordinate system conversion unit that calculates a body surface curve approximating a body surface of the subject based on the subject AR mark in the camera image; an ultrasound probe coordinate system conversion unit that calculates 3D coordinate information of the ultrasound probe based on the ultrasound probe AR mark in the camera image; a 3D processor that calculates a position of the region of interest on the body surface of the subject based on the region of interest, the body surface curve of the subject, and the 3D coordinate information of the ultrasound probe; and an ROI/camera image combining unit that displays a ROI projection mark indicating the position of the region of interest in a superimposed manner on the camera image.

JP7284868B discloses a surgical system comprising an ultrasound probe that is inserted into a body cavity of a subject from a trocar and that transmits and receives ultrasound to and from a target tissue, an endoscope that images the ultrasound probe or a knife as a surgical treatment tool and a surface of a target tissue to obtain an endoscopic image, a position specifying unit that specifies positions of the ultrasound probe and the knife in the endoscopic image, and a controller. In the surgical system, first, the position of the ultrasound probe in the endoscopic image is specified, and then a plurality of combinations, each including the position of the ultrasound probe and an ultrasound tomographic image at the position that is formed by transmitting and receiving the ultrasound with the ultrasound probe to and from the target tissue, are stored in a storage unit. In addition, in a case in which the knife is inserted into the body cavity instead of the ultrasound probe, the position of the knife in the endoscopic image is specified, and the ultrasound tomographic image corresponding to the specified position of the knife is displayed on a display unit.

JP4999012B discloses a robot surgical system comprising a first robot arm that holds an ultrasound probe, a second robot arm that holds a surgical instrument, and a master input device for remotely operating a plurality of robot arms, in which the robot surgical system is operable in a first mode in which the second robot arm is fixed at a predetermined position and the first robot arm is moved in accordance with the operation of the master input device, or a second mode in which the second robot arm is moved in accordance with the operation of the master input device and the first robot arm is moved in accordance with a user command.

### SUMMARY OF THE INVENTION

Ultrasound volume data, which is three-dimensional ultrasound data, may be acquired by transmitting and receiving the ultrasound to and from the target tissue prior to the treatment of the treatment target portion included in the target tissue. According to such ultrasound volume data, for example, it is possible to reconstruct an ultrasound tomographic image of any cross-section including the treatment target portion, and the operator can check the ultrasound tomographic image (reconstructed image) of any cross-section.

Here, since the ultrasound volume data is a set of data indicating the signal intensity of the reflected wave from each position of the target tissue, the ultrasound volume data itself does not include information indicating a location of the treatment target portion. Therefore, in the related art, in order to specify a region of the treatment target portion (including a position, a size, and a shape thereof) in the ultrasound volume data, it is necessary for a human to perform a process of analyzing the ultrasound volume data or the like.

An object of a treatment support apparatus disclosed in the present specification is to easily specify a region of a treatment target portion in ultrasound volume data.

A treatment support apparatus disclosed in the present specification comprises: a three-dimensional model acquisition unit that acquires a three-dimensional model of a target tissue, which is formed based on medical volume data acquired by a medical apparatus and is defined in a model coordinate system, the three-dimensional model having information indicating a region occupied by a treatment target portion of the target tissue; a probe position/orientation detection unit that detects a position and an orientation of an ultrasound probe; a volume data acquisition unit that acquires ultrasound volume data, which is formed based on reception signals obtained by transmitting and receiving ultrasound to and from the target tissue with the ultrasound probe prior to treatment on the treatment target portion, the ultrasound volume data being defined in an ultrasound data coordinate system based on the position and the orientation of the ultrasound probe in a case in which the reception signals are acquired; a coordinate system conversion unit that defines the three-dimensional model in the ultrasound data coordinate system by performing alignment between the three-dimensional model and the ultrasound volume data based on a plurality of feature points included in the three-dimensional model and a plurality of feature points included in the ultrasound volume data; and a treatment target portion specifying unit that specifies a region of a treatment target portion in the ultrasound volume data based on the treatment target portion included in the three-dimensional model defined in the ultrasound data coordinate system.

The ultrasound probe may be held by a robot arm controlled by a robot control device, the ultrasound data coordinate system may correspond to a robot coordinate system which is used by the robot control device to determine a position and an orientation of the robot arm and is further based on a fixed position/orientation relationship between the ultrasound probe and the robot arm that holds the ultrasound probe, and the treatment support apparatus may further comprise: a coordinate system correspondence specifying unit that specifies a correspondence between a camera coordinate system, which is a coordinate system of a camera held by the robot arm controlled by the robot control device, and the robot coordinate system based on a fixed position/orientation relationship between the camera and the robot arm; and a display controller that displays, on a display unit, a target position indicator indicating a surface position of the treatment target portion in the three-dimensional model in a superimposed manner on a captured image for display formed by imaging the target tissue with the camera based on the correspondence between the camera coordinate system and the robot coordinate system.

The ultrasound probe may be held by a robot arm controlled by a robot control device, the ultrasound data coordinate system may correspond to a robot coordinate system which is used by the robot control device to determine a position and an orientation of the robot arm and is further based on a fixed position/orientation relationship between the ultrasound probe and the robot arm that holds the ultrasound probe, and the treatment support apparatus may further comprise: a coordinate system correspondence specifying unit that specifies a correspondence between a camera coordinate system, which is a coordinate system of a camera held by the robot arm controlled by the robot control device, and the robot coordinate system based on a fixed position/orientation relationship between the camera and the robot arm; a treatment tool position/orientation detection unit that specifies a position and an orientation, in the robot coordinate system, of a treatment tool which is used for performing the treatment on the treatment target portion and is held by the robot arm controlled by the robot control device; a current position specifying unit that specifies a current position which is a position on a surface of the target tissue in vicinity of a distal end of the treatment tool in a current position and orientation in the three-dimensional model; and a display controller that displays, on a display unit, a current position indicator indicating the current position in a superimposed manner on a captured image for display formed by imaging the target tissue with the camera based on a correspondence between the camera coordinate system and the ultrasound data coordinate system.

The treatment support apparatus may further comprise: a treatment tool position/orientation detection unit that specifies a position and an orientation, in the robot coordinate system, of a treatment tool which is used for performing the treatment on the treatment target portion and is held by the robot arm controlled by the robot control device; and a current position specifying unit that specifies a current position that is a position on a surface of the target tissue in vicinity of a distal end of the treatment tool in a current position and orientation in the three-dimensional model, in which the display controller further displays a current position indicator indicating the current position in a superimposed manner on the captured image for display based on a correspondence between the camera coordinate system and the robot coordinate system.

The treatment support apparatus may further comprise: a rate-of-match calculation unit that calculates a rate of match between the surface position of the treatment target portion and the current position of the treatment tool; and a notification controller that notifies an operator in a case in which the rate of match is equal to or greater than a rate-of-match threshold value.

The target position indicator may include scale gradations based on dimensions in a real space in vicinity of the surface position of the treatment target portion.

The current position indicator may include scale gradations based on dimensions in a real space in vicinity of the current position.

The treatment support apparatus may further comprise: a reconstruction processing unit that specifies a reconstructed cross-section based on a position and an orientation of the treatment tool in the robot coordinate system and reconstructs the ultrasound volume data to form a two-dimensional reconstructed image representing the reconstructed cross-section, in which the display controller displays the two-dimensional reconstructed image on a display unit and displays a reconstructed image interior target position indicator indicating the treatment target portion in the reconstructed cross-section in a superimposed manner on the two-dimensional reconstructed image.

The treatment support apparatus may further comprise: a model cross-section image formation unit that specifies a model cut cross-section based on a position and an orientation of the treatment tool in the robot coordinate system and forms a model cross-section image representing the model cut cross-section of at least one of the three-dimensional models before the treatment target portion is removed or after the treatment target portion is removed, in which the display controller displays the model cross-section image on a display unit.

The display controller may display the model cross-section image on the display unit and display a model cross-section image interior target position indicator indicating the treatment target portion in a superimposed manner on the model cross-section image.

In addition, a treatment support program disclosed in the present specification causes a computer to function as: a three-dimensional model acquisition unit that acquires a three-dimensional model of a target tissue, which is formed based on medical volume data acquired by a medical apparatus and is defined in a model coordinate system, the three-dimensional model having information indicating a region occupied by a treatment target portion of the target tissue; a probe position/orientation detection unit that detects a position and an orientation of an ultrasound probe; a volume data acquisition unit that acquires ultrasound volume data, which is formed based on reception signals obtained by transmitting and receiving ultrasound to and from the target tissue with the ultrasound probe prior to treatment on the treatment target portion, the ultrasound volume data being defined in an ultrasound data coordinate system based on the position and the orientation of the ultrasound probe in a case in which the reception signals are acquired; a coordinate system conversion unit that defines the three-dimensional model in the ultrasound data coordinate system by performing alignment between the three-dimensional model and the ultrasound volume data based on a plurality of feature points included in the three-dimensional model and a plurality of feature points included in the ultrasound volume data; and a treatment target portion specifying unit that specifies a region of a treatment target portion in the ultrasound volume data based on the treatment target portion included in the three-dimensional model defined in the ultrasound data coordinate system.

With the treatment support apparatus disclosed in the present specification, it is possible to easily specify the region of the treatment target portion in the ultrasound volume data.

### BRIEF DESCRIPTION OF THE DRAWINGS

FIG. 1 is a schematic configuration diagram of a treatment support system according to the present embodiment.
FIG. 2 is a conceptual diagram showing an endoscope camera, an ultrasound probe, and a target tissue.
FIG. 3 is a conceptual diagram showing the endoscope camera, a treatment tool, and the target tissue.
FIG. 4 is a schematic configuration diagram of an ultrasound diagnostic apparatus according to the present embodiment.
FIG. 5 is a view showing an example of a three-dimensional model of the target tissue.
FIG. 6 is a view showing an example of a captured image for detection.
FIG. 7 is a conceptual diagram showing a concept of a volume data formation process.
FIG. 8 is a conceptual diagram showing a concept of alignment between the three-dimensional model and ultrasound volume data.
FIG. 9 is a view showing a first display example of a captured image for display.
FIG. 10 is a view showing a second display example of the captured image for display.
FIG. 11 is a conceptual diagram showing an aspect of a process of specifying a current position in the three-dimensional model.
FIG. 12 is a view showing a display example of a current position indicator.
FIG. 13 is a view showing an example of notification that a rate of match between a surface position of a treatment target portion and the current position is equal to or greater than a rate-of-match threshold value.
FIG. 14 is a conceptual diagram showing a concept of a process of reconstructing the ultrasound volume data.
FIG. 15 is a view showing a display example of a two-dimensional reconstructed image.
FIG. 16 is a conceptual diagram showing a concept of a process of forming a model cross-section image.
FIG. 17 is a view showing a display example of the model cross-section image.
FIG. 18 is a first flowchart showing a flow of a process of the ultrasound diagnostic apparatus according to the present embodiment.
FIG. 19 is a second flowchart showing a flow of a process of the ultrasound diagnostic apparatus according to the present embodiment.

### DESCRIPTION OF THE PREFERRED EMBODIMENTS

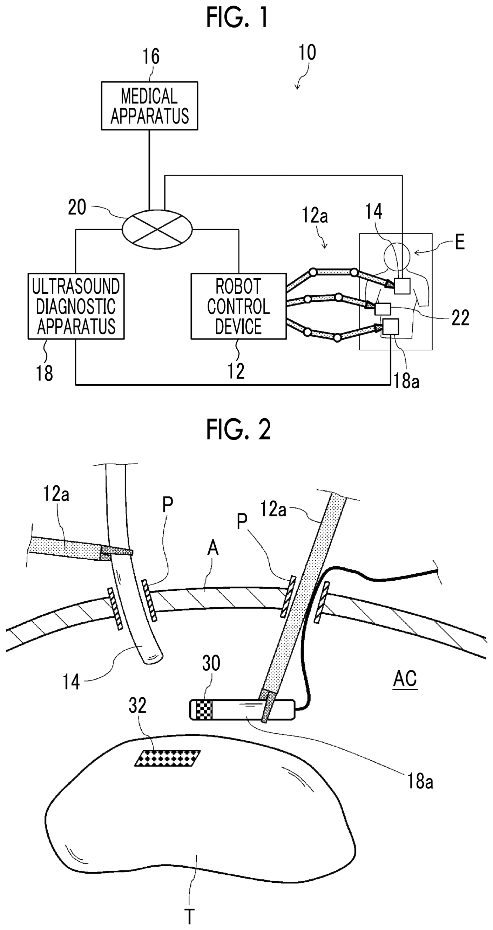

FIG. 1 is a schematic configuration diagram of a treatment support system 10 according to the present embodiment. The treatment support system 10 includes a robot control device 12 including a plurality of robot arms 12a, an endoscope 14, a medical apparatus 16, and an ultrasound diagnostic apparatus 18 as a treatment support apparatus including an ultrasound probe 18a. The robot control device 12, the endoscope 14, the medical apparatus 16, and the ultrasound diagnostic apparatus 18 are connected to each other via a communication line 20 such as a wide area network (WAN) or a local area network (LAN) so as to be able to communicate with each other.

The robot control device 12 includes, in addition to the plurality of robot arms 12a, a processor configured by a central processing unit (CPU) and the like, a communication interface configured by a network adapter and the like, and an input interface configured by a surgeon console and the like. The processor of the robot control device 12 controls the robot arm 12a in accordance with an instruction from an operator such as a doctor. The operator may directly input the instruction to the robot control device 12 through the input interface of the robot control device 12, or may remotely input the instruction through the communication line 20.

The robot arm 12a holds the endoscope 14, the ultrasound probe 18a, and a treatment tool 22 (for example, scissors or forceps) for the treatment. That is, the robot control device 12 controls a position and an orientation of the endoscope 14, a position and an orientation of the ultrasound probe 18a, and a position and an orientation of the treatment tool 22.

The endoscope 14 includes, in addition to a lens and an image sensor, a processor configured by a CPU and the like, a communication interface configured by a network adapter and the like, and the like. The endoscope 14 is a camera that images the inside of the body cavity of the subject E. An endoscopic image is formed by the image sensor of the endoscope 14, and the endoscopic image is transmitted to the ultrasound diagnostic apparatus 18 through the communication interface of the endoscope 14. In addition, the endoscope 14 may be directly connected to the ultrasound diagnostic apparatus 18 through a cable or the like without going through the communication line 20, and the endoscopic image may be directly transmitted to the ultrasound diagnostic apparatus 18.

FIG. 2 is a conceptual diagram showing the endoscope 14, the ultrasound probe 18a, and a target tissue T (organ in the example of FIG. 2). In the present embodiment, it is assumed that laparoscopic surgery is performed on the subject E. FIG. 2 shows a state in which the ultrasound probe 18a is inserted into an abdominal cavity AC prior to the laparoscopic surgery. A small hole is made in an abdomen A of the subject E, and a sleeve (port) P is attached to the hole. The abdominal cavity AC is inflated with gas, and the endoscope 14 and the ultrasound probe 18a are inserted into the abdominal cavity AC from the sleeve P. In the present embodiment, the ultrasound probe 18a is a drop-in type probe that is inserted into the body cavity (in the example of FIG. 2, the abdominal cavity). As described above, the endoscope 14 and the ultrasound probe 18a are held by the robot arm 12a, and the robot control device 12 controls the positions and the orientations of the endoscope 14 and the ultrasound probe 18a.

A probe detection mark 30 is attached to the ultrasound probe 18a. The probe detection mark 30 is a mark for detecting the position and the orientation of the ultrasound probe 18a. The position and the orientation of the ultrasound probe 18a can be detected by imaging the probe detection mark 30 with the endoscope 14 to acquire an endoscopic image, and analyzing an image of the probe detection mark 30 shown in the endoscopic image. Examples of the probe detection mark 30 include an augmented reality (AR) marker.

In addition, a target detection mark 32 is attached to a surface of the target tissue T. The target detection mark 32 is a mark for detecting the position and the orientation of the target tissue T (surface of the target tissue T). The position and the orientation of the target detection mark 32 can be detected by imaging the target detection mark 32 with the endoscope 14 to acquire the endoscopic image, and analyzing an image of the target detection mark 32 shown in the endoscopic image. Examples of the target detection mark 32 include an AR marker. The target detection mark 32 has a different pattern from the probe detection mark 30.

Although details will be described later, in the present embodiment, before the treatment on the target tissue T, the ultrasound is transmitted and received to and from the target tissue T, and the ultrasound volume data representing the target tissue T is formed based on a reception signal obtained by the transmission and reception.

In the present specification, a case in which the ultrasound is transmitted and received to and from the target tissue T for forming the ultrasound volume data in this way is expressed as "ultrasound transmission and reception".

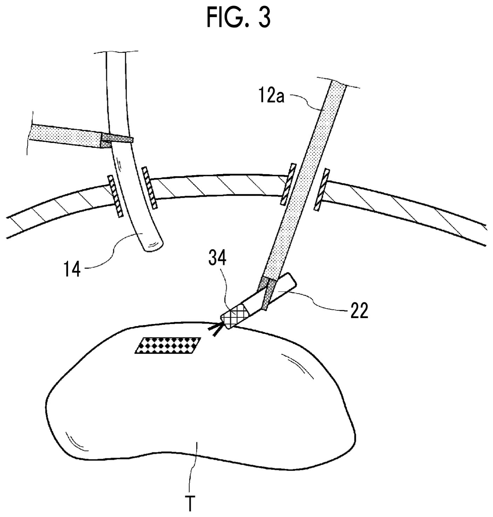

FIG. 3 is a conceptual diagram showing the endoscope 14, the treatment tool 22, and the target tissue T. As shown in FIG. 3, in a case in which the treatment on the target tissue T (particularly, a treatment target portion thereof) is performed, the treatment tool 22 is held by the robot arm 12a instead of the ultrasound probe 18a. Then, the treatment tool 22 is inserted into the abdominal cavity AC from the sleeve P, and the operator controls the robot arm 12a to perform the treatment on the target tissue T using the treatment tool 22. In FIG. 3, the treatment tool 22 is held by the robot arm 12a, but the robot arm distal end part may be provided with the treatment tool 22 such as forceps or an excision tool. In this case, since the robot coordinate system is used as the basis in advance, the treatment tool detection mark 34 is not necessary.

A treatment tool detection mark 34 is attached to the treatment tool 22. The treatment tool detection mark 34 is a mark for detecting the position and the orientation of the treatment tool 22. The position and the orientation of the treatment tool 22 can be detected by imaging the treatment tool detection mark 34 with the endoscope 14 to acquire the endoscopic image, and analyzing an image of the treatment tool detection mark 34 shown in the endoscopic image. Examples of the treatment tool detection mark 34 include an AR marker. The treatment tool detection mark 34 has a different pattern from the probe detection mark 30 and the target detection mark 32.

In the present specification, a period before the treatment on the target tissue T is performed by the treatment tool 22, but, in a case in which the treatment on the target tissue T is about to be performed (in other words, after the ultrasound volume data is formed and before the treatment on the target tissue T is performed), and a period from the start of the treatment on the target tissue T to the completion of the treatment are expressed as "during the treatment on the target tissue T".

A procedure of the treatment on the target tissue T will be described here. First, during the ultrasound transmission and reception, the ultrasound is transmitted to and received from the target tissue T by the ultrasound probe 18a inserted into the abdominal cavity AC. In this manner, an ultrasound tomographic image is formed in the ultrasound diagnostic apparatus 18. In the present embodiment, a plurality of ultrasound tomographic images are formed by transmitting and receiving the ultrasound while moving the ultrasound probe 18a in a direction substantially perpendicular to an ultrasound transmission/reception surface. The ultrasound diagnostic apparatus 18 forms the ultrasound volume data, which is three-dimensional ultrasound data, from the plurality of ultrasound tomographic images. During the ultrasound transmission and reception, the endoscope 14 images the probe detection mark 30 and the target detection mark 32 to form the endoscopic image.

In the present specification, the endoscopic image is referred to as a "captured image for detection". The captured image for detection includes the image of the probe detection mark 30 and the image of the target detection mark 32. In particular, the image of the probe detection mark 30 included in the captured image for detection is information indicating the position and the orientation of the ultrasound probe 18a during the ultrasound transmission and reception, and the image of the target detection mark 32 included in the captured image for detection is information indicating the position and the orientation of the target tissue T (specifically, the surface thereof) during the ultrasound transmission and reception. The captured image for detection is transmitted from the endoscope 14 to the ultrasound diagnostic apparatus 18.

Then, the treatment on the target tissue T is started by the operator. Even in a case of the treatment on the target tissue T, the target tissue T is imaged by the endoscope 14. In the present specification, the endoscopic image formed by the endoscope 14 during the treatment on the target tissue T is referred to as a "captured image for display". In order to perform the treatment on the target tissue T, the treatment tool 22 is held by the robot arm 12a instead of the ultrasound probe 18a, and the treatment tool 22 is inserted into the abdominal cavity AC. The captured image for display may include the image of the treatment tool 22 (specifically, the treatment tool detection mark 34) and the image of the target detection mark 32. In particular, the image of the treatment tool detection mark 34 included in the captured image for display is information indicating the position and the orientation of the treatment tool 22 during the treatment on the target tissue T (in other words, the current time), and the image of the target detection mark 32 included in the captured image for display is information indicating the position and the orientation of the target tissue T during the treatment on the target tissue T. The captured image for display is transmitted from the endoscope 14 to the ultrasound diagnostic apparatus 18.

Here, the treatment target portion (for example, a tumor) of the target tissue T may be located at a position that is not visible from the surface of the target tissue T. In this case, the operator confirms the position or the shape of the treatment target portion inside the target tissue T based on the ultrasound volume data formed during the ultrasound transmission and reception. Then, the position on the surface of the target tissue T that should be treated with the treatment tool 22 is determined. The treatment support system 10 according to the present embodiment supports the operator in this regard.

The medical apparatus 16 is an apparatus that acquires medical volume data of the target tissue T of the subject E acquired by a medical apparatus other than the ultrasound diagnostic apparatus. The medical apparatus 16 may be a medical data management server that receives and manages the medical volume data from the medical apparatus other than the ultrasound diagnostic apparatus, or may be the medical apparatus itself that forms the medical volume data other than the ultrasound diagnostic apparatus. The medical volume data is, for example, three-dimensional computed tomography (CT) data formed by a CT apparatus as the medical apparatus or three-dimensional magnetic resonance imaging (MRI) data formed by an MRI apparatus as the medical apparatus. The medical volume data is data in which voxels each having data are arranged in three dimensions. The medical volume data has a coordinate value indicating a position of each voxel in a medical data coordinate system. That is, if the medical volume data includes the treatment target portion, the medical volume data has information indicating a region occupied by the treatment target portion. The medical apparatus 16 acquires the medical volume data of the target tissue T and transmits the medical volume data to the ultrasound diagnostic apparatus 18 prior to the ultrasound transmission and reception (that is, the ultrasound transmission and reception) to and from the target tissue T and the treatment on the target tissue T.

The medical apparatus may be an ultrasound diagnostic apparatus, and the medical volume data may be ultrasound volume data formed by the ultrasound diagnostic apparatus. As described above, the ultrasound volume data itself does not include information indicating the location of the treatment target portion, but information indicating the region occupied by the treatment target portion is added to the ultrasound volume data serving as the medical volume data through analysis of the ultrasound volume data by a human. Further, information indicating a position of a feature point for alignment to be described later is added to the ultrasound volume data through analysis of the ultrasound volume data by a human.

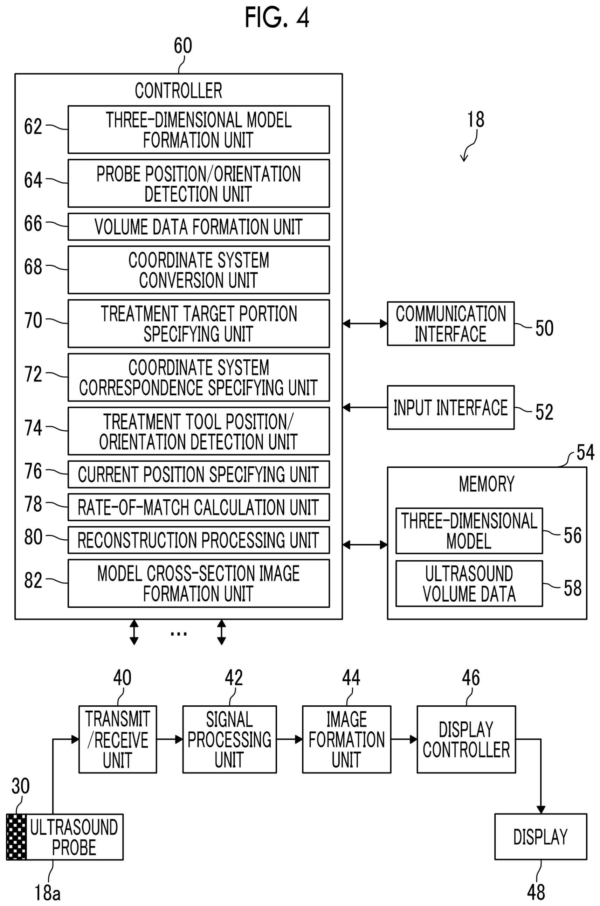

FIG. 4 is a schematic configuration diagram of the ultrasound diagnostic apparatus 18. The ultrasound diagnostic apparatus 18 is a medical apparatus installed in medical institutions such as a hospital.

The ultrasound probe 18a is a device that transmits and receives the ultrasound to and from the target tissue T of the subject E. The ultrasound probe 18a includes a transducer array consisting of a plurality of transducer elements that transmit and receive the ultrasound to and from the target tissue T. In the ultrasound probe 18a, the transducer array is formed of the plurality of transducer elements arranged in one row. In a case in which a transmission signal is supplied to each transducer element from a transmit/receive unit 40, which will be described later, each transducer element generates the ultrasound.

As descried above, the probe detection mark 30 is attached to the ultrasound probe 18a.

The transmit/receive unit 40 transmits the transmission signal to the ultrasound probe 18a (specifically, each transducer element of the transducer array) under control of a controller 60 described later. In addition, the transmit/receive unit 40 receives the reception signal from each transducer element that has received the reflected wave reflected by the target tissue T. The transmit/receive unit 40 includes an adder and a plurality of delay elements corresponding to the respective transducer elements, and performs, using the adder and the plurality of delay elements, phase-aligned summation (delay-and-sum) processing in which the reception signals from the respective transducer elements are phase-aligned and added. As a result, a reception beam signal is formed in which pieces of information indicating a signal intensity of the reflected wave reflected by the target tissue T are arranged in a depth direction of the target tissue T.

The signal processing unit 42 executes various kinds of signal processing including filter processing of applying a bandpass filter, detection processing, and the like, on the reception beam signal from the transmit/receive unit 40.

The image formation unit 44 forms the ultrasound tomographic image (B-mode image) representing the cross-section (particularly, the ultrasound transmission/reception surface) of the target tissue T based on the reception beam signal on which the signal processing has been performed by the signal processing unit 42.

The display controller 46 performs control to display various images including the ultrasound tomographic image formed by the image formation unit 44 on a display 48.

The display 48 as a display unit is, for example, a display device configured by a liquid crystal display, an organic electroluminescence (EL), or the like.

The transmit/receive unit 40, the signal processing unit 42, the image formation unit 44, and the display controller 46 provided in the ultrasound diagnostic apparatus 18 are configured by a processor. The processor includes at least one of a general-purpose processing device (for example, a CPU) or a dedicated processing device (for example, a graphics processing unit (GPU), an application-specific integrated circuit (ASIC), a field-programmable gate array (FPGA), or a programmable logic device). The processor need not be configured by a single processing device but rather may be configured by cooperation of a plurality of processing devices present at physically separated positions. Further, each of the above-described units may be implemented by cooperation of hardware such as a processor, and software.

A communication interface 50 is configured by, for example, a network adapter. The communication interface 50 exhibits a function of communicating with other devices (particularly, the robot control device 12, the endoscope 14, and the medical apparatus 16) via the communication line 20. In particular, the communication interface 50 receives the endoscopic image from the endoscope 14, receives position/orientation information indicating the position and the orientation of each robot arm 12a from the robot control device 12, and receives the medical volume data from the medical apparatus 16.

An input interface 52 is configured by, for example, a button, a trackball, or a touch panel. The input interface 52 is used to input an instruction of the operator, who uses the ultrasound diagnostic apparatus 18, to the ultrasound diagnostic apparatus 18.

A memory 54 includes a hard disk drive (HDD), a solid-state drive (SSD), an embedded Multi Media Card (eMMC), a read-only memory (ROM), a random-access memory (RAM), or the like. The memory 54 stores a treatment support program for operating each unit of the ultrasound diagnostic apparatus 18. The treatment support program can also be stored in a non-transitory computer-readable storage medium such as a Universal Serial Bus (USB) memory or a CD-ROM. The ultrasound diagnostic apparatus 18 can read the treatment support program from such a storage medium to execute the treatment support program. Since the ultrasound diagnostic apparatus 18 reads the treatment support program to exhibit functions described later, the ultrasound diagnostic apparatus 18 can be said to be a computer program product.

In addition, as shown in FIG. 4, a three-dimensional model 56 and ultrasound volume data 58 are stored in the memory 54. The three-dimensional model 56 is formed by a three-dimensional model formation unit 62 described later. In addition, the ultrasound volume data 58 is formed by a volume data formation unit 66, which will be described later, based on the reception signals obtained by the ultrasound transmission and reception to and from the target tissue T. Details of the three-dimensional model 56 and the ultrasound volume data 58 will be described later.

The controller 60 includes at least one of a general-purpose processor (such as a CPU) or a dedicated processor (such as a GPU, an ASIC, an FPGA, or a programmable logic device). The controller 60 need not be configured by a single processing device but may be configured by cooperation of a plurality of processing devices present at physically separated positions. The controller 60 controls the units of the ultrasound diagnostic apparatus 18. In addition, the controller 60 exhibits the functions as the units shown in FIG. 4 in accordance with the treatment support program stored in the memory 54. Hereinafter, the details of the functions of the units exhibited by the controller 60 will be described.

The three-dimensional model formation unit 62 as a three-dimensional model acquisition unit forms the three-dimensional model of the target tissue T based on the medical volume data received from the medical apparatus 16.

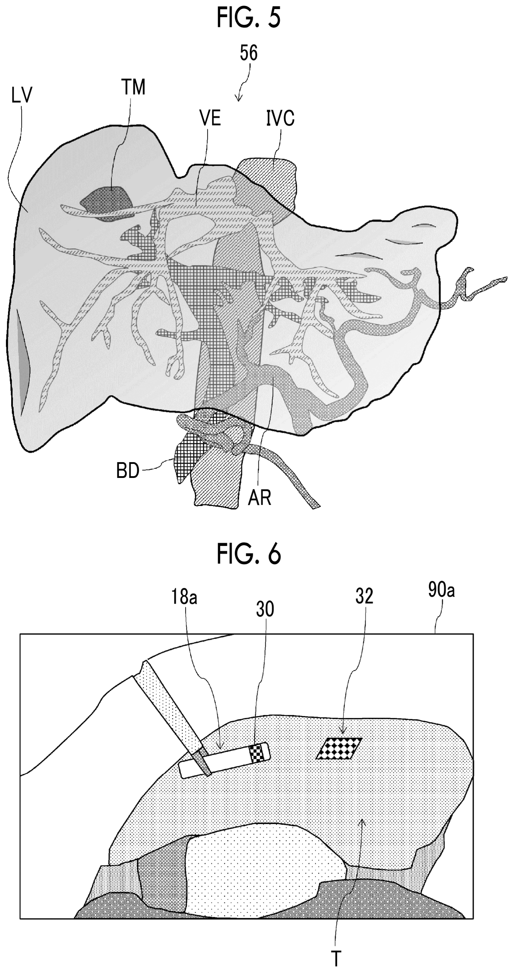

FIG. 5 is a view showing an example of the three-dimensional model 56 of the target tissue T. In the example of FIG. 5, the three-dimensional model 56 is formed from medical volume data that include the liver as the target tissue T and surrounding tissues such as the inferior vena cava, veins, arteries, bile ducts, and tumors. In the present embodiment, it is assumed that the tumor has developed in the liver and that the tumor is the treatment target portion. The three-dimensional model 56 includes a liver model LV, an inferior vena cava model IVC, a vein model VE, an artery model AR, a bile duct model BD, and a tumor model TM. In principle, the three-dimensional model 56 representing the liver and the surroundings thereof also includes models of a portal vein, a vascular region, and other tissues, but these are not shown in the three-dimensional model 56.

Since a known method can be used as a method of forming the three-dimensional model 56 based on the medical volume data, detailed description thereof will be omitted here, but the three-dimensional model formation unit 62 forms the three-dimensional model 56 using a technique such as volume rendering or surface rendering.

In a case in which the medical volume data has the position information of each voxel, the three-dimensional model 56 is formed from the medical volume data, and thus the three-dimensional model 56 also has the position information (coordinates) indicating each position. The position information included in the three-dimensional model 56 is information indicating a position in a model coordinate system (the same coordinate system as the medical data coordinate system).

In addition, the three-dimensional model 56 has attribute information on each tissue including the tumor of the target tissue T. For example, the three-dimensional model 56 has information such as a name, a volume, and a diameter of each tissue in association with the model of the tissue. These pieces of information may be set by automatic calculation in a case in which the three-dimensional model formation unit 62 forms the three-dimensional model 56, or may be set manually by the operator or the like. That is, the three-dimensional model 56 has information indicating a region occupied by the treatment target portion (in the present embodiment, the tumor) in the target tissue T (in the present embodiment, the liver). Specifically, the three-dimensional model 56 has information indicating a coordinate region (may be referred to as a position and a size of the tumor) occupied by the tumor that is the treatment target portion in the model coordinate system. When the medical volume data is the ultrasound volume data also, because the ultrasound volume data serving as the medical volume data includes information indicating the region occupied by the tumor that is the treatment target portion as described above, the three-dimensional model 56 formed from the ultrasound volume data includes information indicating the coordinate region occupied by the tumor that is the treatment target portion in the model coordinate system.

In addition, the three-dimensional model 56 may be formed by the medical apparatus 16, and the ultrasound diagnostic apparatus 18 may receive the three-dimensional model 56 from the medical apparatus 16. In such a case, the communication interface 50 exhibits the function as the three-dimensional model acquisition unit, and the controller 60 need not exhibit the function as the three-dimensional model formation unit 62.

The probe position/orientation detection unit 64 detects the position and the orientation of the ultrasound probe 18a. In the present embodiment, the probe position/orientation detection unit 64 detects the position and the orientation of the ultrasound probe 18a during the ultrasound transmission and reception based on the captured image for detection formed by the endoscope 14. In the present embodiment, the ultrasound volume data 58 is composed of the plurality of ultrasound tomographic images, and the probe position/orientation detection unit 64 detects the position and the orientation of the ultrasound probe 18a at a timing at which each ultrasound tomographic image forming the ultrasound volume data 58 is formed (in other words, during the ultrasound transmission and reception for the ultrasound tomographic image).

FIG. 6 is a view showing an example of a captured image for detection 90a. As described above, the captured image for detection 90a includes the image of the probe detection mark 30 indicating the position and the orientation of the ultrasound probe 18a during the ultrasound transmission and reception. The probe position/orientation detection unit 64 detects the position and the orientation of the ultrasound probe 18a during the ultrasound transmission and reception in a camera coordinate system of the endoscope 14 by analyzing the image of the probe detection mark 30 in the captured image for detection 90a. The camera coordinate system is a coordinate system in which the position of the endoscope 14 is an origin, and is, for example, a coordinate system defined by three axes in which a direction of an optical axis of a lens of the endoscope 14 is a z axis, a direction perpendicular to the z axis is an x axis, and a direction perpendicular to the x axis and the z axis is a y axis. Since a known method can be used as a method of detecting the position and the orientation of the ultrasound probe 18a in the camera coordinate system from the image of the probe detection mark 30 included in the captured image for detection 90a, a detailed description thereof will be omitted here.

Next, the probe position/orientation detection unit 64 converts position/orientation information (hereinafter, referred to as "probe position/orientation information") indicating the position and the orientation of the ultrasound probe 18a in the camera coordinate system into position/orientation information in a robot coordinate system. The robot coordinate system is a coordinate system that is recognized by the robot control device 12 and that is used to represent the position and the orientation of the robot arm 12a.

Since the ultrasound probe 18a is held by the robot arm 12a, a position/orientation relationship between the ultrasound probe 18a and the robot arm 12a is fixed (not changed). In the present specification, a fixed position/orientation relationship between the ultrasound probe 18a and the robot arm 12a is referred to as "arm-probe position/orientation relationship". The arm-probe position/orientation relationship can be, for example, represented by a vector in the robot coordinate system. The probe position/orientation detection unit 64 converts the probe position/orientation information in the camera coordinate system into the position/orientation information in the robot coordinate system in accordance with an arm-probe position/orientation relationship.

Specifically, the probe position/orientation detection unit 64 acquires the position/orientation information of the robot arm 12a that holds the ultrasound probe 18a in the robot coordinate system and the information indicating the arm-probe position/orientation relationship from the robot control device 12. The information indicating the arm-probe position/orientation relationship may be input to the robot control device 12 by, for example, the operator who causes the robot arm 12a to hold the ultrasound probe 18a. The probe position/orientation detection unit 64 can obtain the probe position/orientation information in the robot coordinate system by using the position/orientation information of the robot arm 12a in the robot coordinate system and the arm-probe position/orientation relationship. Here, by aligning the respective axial directions of the camera coordinate system and the robot coordinate system, a transformation vector from the camera coordinate system to the robot coordinate system can be obtained based on the probe position/orientation information in the camera coordinate system and the robot coordinate system. The probe position/orientation detection unit 64 converts the probe position/orientation information in the camera coordinate system into the position/orientation information in the robot coordinate system by using the transformation vector.

The probe position/orientation detection unit 64 can also obtain the probe position/orientation information in the robot coordinate system by simply using the position/orientation information of the robot arm 12a in the robot coordinate system and the arm-probe position/orientation relationship without analyzing the image of the probe detection mark 30 in the captured image for detection 90a. However, the position and the orientation of the ultrasound probe 18a can be detected with higher accuracy in a case in which the position and the orientation are detected by using the probe detection mark 30.

The probe position/orientation detection unit 64 stores the probe position/orientation information in the memory 54 in association with each ultrasound tomographic image in a case in which the ultrasound tomographic image is formed.

The probe position/orientation detection unit 64 may further detect the position and the orientation of the target tissue T during the ultrasound transmission and reception.

As described above, the captured image for detection 90a also includes the image of the target detection mark 32 indicating the position and the orientation of the target tissue T during the ultrasound transmission and reception. The probe position/orientation detection unit 64 detects the position and the orientation of the target tissue T during the ultrasound transmission and reception in the camera coordinate system of the endoscope 14 by analyzing the image of the target detection mark 32 in the captured image for detection 90a. Since a known method can be used as a method of detecting the position and the orientation of the target tissue T in the camera coordinate system from the image of the target detection mark 32 included in the captured image for detection 90a, a detailed description thereof will be omitted here. In addition, the probe position/orientation detection unit 64 may detect the position and orientation of the ultrasound probe 18a relative to the position and orientation of the target tissue T (the surface thereof). As a result, the position and the orientation of the ultrasound probe 18a relative to the target tissue T, which absorb any variation in the position or the orientation of the target tissue T, can be obtained.

Next, similarly to the ultrasound probe 18a, the probe position/orientation detection unit 64 converts the position/orientation information indicating the position and the orientation of the target tissue T in the camera coordinate system into the position/orientation information in the robot coordinate system in accordance with the arm-probe position/orientation relationship. In the present specification, information indicating the position and the orientation of the target tissue T detected based on the captured image for detection 90a is referred to as target position/orientation information during transmission and reception. The probe position/orientation detection unit 64 stores the target position/orientation information during transmission and reception in the memory 54 in association with each ultrasound tomographic image.

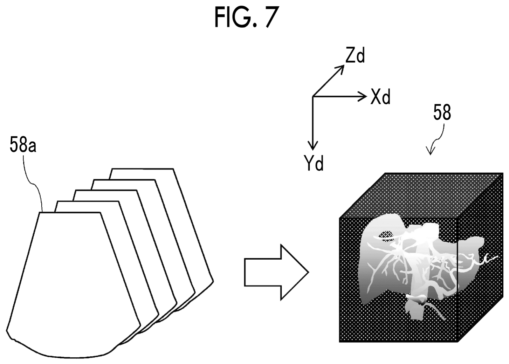

FIG. 7 is a conceptual diagram showing a concept of a volume data formation process performed by the volume data formation unit 66. The volume data formation unit 66 as a volume data acquisition unit forms the ultrasound volume data 58 based on the reception signals obtained by transmitting and receiving the ultrasound to and from the target tissue T with the ultrasound probe 18a prior to the treatment on the treatment target portion of the target tissue T. The ultrasound volume data 58 is data in which the voxels each having data (signal intensity of reflected wave from the subject E and the like) are three-dimensionally arranged.

In the present embodiment, the volume data formation unit 66 forms the ultrasound volume data 58 based on a plurality of ultrasound tomographic images 58a as the reception signals. The ultrasound volume data 58 is formed during treatment of the target tissue T, more specifically, during transmission and reception of the ultrasound described above, and differs from the ultrasound volume data serving as the medical volume data which is acquired in advance. Since a known method can be used as a method of forming the ultrasound volume data 58 based on the plurality of ultrasound tomographic images 58a, a detailed description thereof will be omitted here. As described above, since the ultrasound volume data 58 is a set of the voxels each having data, the reception signals for forming the ultrasound volume data 58 need not necessarily be the plurality of ultrasound tomographic images 58a. For example, a plurality of frame data (a set of two-dimensional arrayed data corresponding to one ultrasound tomographic image 58a) before the image formation may be used.

As described above, the probe position/orientation information (in the present embodiment, in the robot coordinate system) at the time of forming the ultrasound tomographic image 58a is associated with each ultrasound tomographic image 58a. Each position (coordinate) in the ultrasound tomographic image 58a can be specified based on the position and the orientation of the ultrasound probe 18a in a case in which the ultrasound tomographic image 58a is formed. That is, it can be said that coordinate information indicating each position is attached to each ultrasound tomographic image 58a. Therefore, the ultrasound volume data 58 composed of the plurality of ultrasound tomographic images 58a also has coordinate information indicating each position. In the present embodiment, the coordinate information of the ultrasound volume data 58 is coordinate information in the robot coordinate system (represented by an Xd axis, a Yd axis, and a Zd axis in FIG. 7) as the ultrasound data coordinate system based on the position and the orientation of the ultrasound probe 18a in a case in which the reception signal corresponding to each ultrasound tomographic image 58a is acquired.

In the present embodiment, the ultrasound volume data 58 is composed of the plurality of ultrasound tomographic images 58a, but, in a case in which a 2D array probe in which the transducer elements are arranged in a two-dimensional manner is used as the ultrasound probe 18a, the volume data formation unit 66 may directly form the ultrasound volume data 58 based on three-dimensional reception signals from the ultrasound probe 18a. Even in this case, each position (coordinate) in the ultrasound volume data 58 can be specified based on the position and the orientation of the ultrasound probe 18a in a case in which the ultrasound volume data 58 is formed. That is, even in this case, the coordinate information indicating each position of the ultrasound volume data 58 in the robot coordinate system is added to the ultrasound volume data 58.

The coordinate system conversion unit 68 defines the three-dimensional model 56 defined in the model coordinate system (in other words, the position of each voxel is represented by the coordinates in the model coordinate system) in the same robot coordinate system as the ultrasound volume data 58. That is, the coordinate system conversion unit 68 converts the coordinates of each voxel of the three-dimensional model 56 in the model coordinate system into the coordinates in the robot coordinate system.

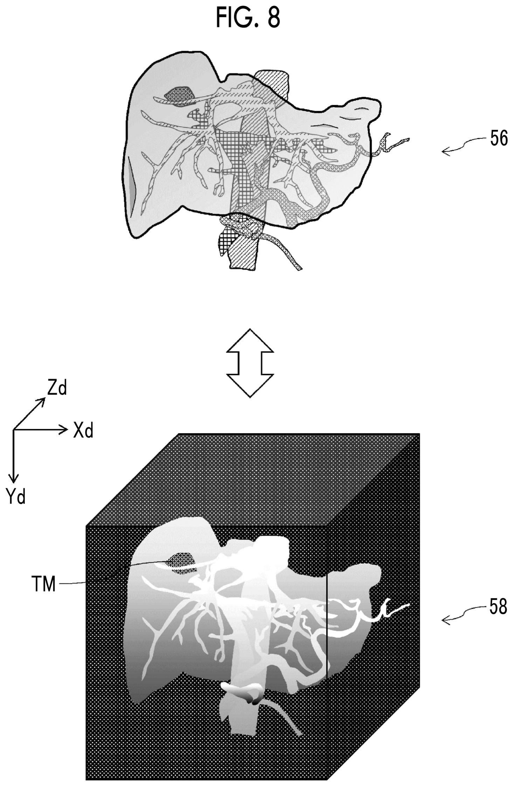

FIG. 8 is a conceptual diagram showing a concept of alignment between the three-dimensional model 56 and the ultrasound volume data 58. Specifically, first, the coordinate system conversion unit 68 specifies the positions of a plurality of feature points in the three-dimensional model 56. For example, in a case of the three-dimensional model 56 including the liver and the surrounding tissues thereof, a plurality of points representing a shape of the portal vein are specified as the plurality of feature points. The entire portal vein included in the three-dimensional model 56 may be specified as the feature point. As described above, since the three-dimensional model 56 has the attribute information on each tissue, it is easy to specify the plurality of feature points in the three-dimensional model 56. Further, even in the case where the three-dimensional model 56 is formed from the ultrasound volume data serving as the medical volume data, the three-dimensional model 56 has feature points added to the ultrasound volume data.

Next, the coordinate system conversion unit 68 specifies a plurality of feature points (in the above example, the plurality of feature points representing the shape of the portal vein) corresponding to the plurality of feature points specified in the three-dimensional model 56 in the ultrasound volume data 58. As a method of specifying the plurality of feature points in the ultrasound volume data 58, for example, a learning model that has been trained to extract a specific feature point using the ultrasound volume data as input can be used. Alternatively, the display controller 46 may display the ultrasound volume data 58 on the display 48, and the operator may input the command to designate the plurality of feature points from the input interface 52.

The coordinate system conversion unit 68 performs alignment between the three-dimensional model 56 and the ultrasound volume data 58 based on the plurality of feature points included in the three-dimensional model 56 and the plurality of feature points included in the ultrasound volume data 58, which are specified as described above. Specifically, at least one of the three-dimensional model 56 or the ultrasound volume data 58 is subjected to translation or scaling (enlargement/reduction) such that the plurality of feature points included in the three-dimensional model 56 and the plurality of feature points included in the ultrasound volume data 58 exactly match each other. In a case in which the three-dimensional model 56 and the ultrasound volume data 58 are aligned, it is equivalent to the three-dimensional model 56 being defined in the robot coordinate system. Specifically, the coordinate system conversion unit 68 may set the coordinates of each voxel of the three-dimensional model 56 to the coordinates (the coordinates of each of the Xd axis, the Yd axis, and the Zd axis) of each corresponding voxel of the aligned ultrasound volume data 58. As described above, the coordinate system conversion unit 68 defines the three-dimensional model 56 in the robot coordinate system.

A treatment target portion specifying unit 70 specifies the treatment target portion in the ultrasound volume data 58 based on the treatment target portion included in the three-dimensional model 56 defined in the robot coordinate system. As described above, the three-dimensional model 56 has information indicating the region occupied by the treatment target portion (in the present embodiment, the tumor). The three-dimensional model 56 is defined in the same robot coordinate system as the ultrasound volume data 58 by the coordinate system conversion unit 68. Accordingly, the treatment target portion specifying unit 70 can specify that the same coordinate region as the region occupied by the treatment target portion in the three-dimensional model 56 is the treatment target portion in the ultrasound volume data 58.

As described above, according to the present embodiment, it is possible to easily specify the region of the treatment target portion in the ultrasound volume data 58. In particular, according to the present embodiment, it is possible to specify the position of the treatment target portion in the ultrasound volume data 58 quickly (in real time during a surgery). According to the present embodiment, the region of the treatment target portion can be specified in the ultrasound volume data 58 more quickly in comparison to at least the case where the region of the treatment target portion is specified by a human analyzing the ultrasound volume data 58. By specifying the region of the treatment target portion in the ultrasound volume data 58, it is possible to support the operator who performs the treatment on the treatment target portion, as will be described later.

The coordinate system correspondence specifying unit 72 specifies a correspondence between the camera coordinate system of the endoscope 14 and the robot coordinate system. Since the endoscope 14 is held by the robot arm 12a, a position/orientation relationship between the endoscope 14 and the robot arm 12a is fixed (not changed). Therefore, the position (that is, an origin of the camera coordinate system) and the orientation (that is, the three-axis directions of the camera coordinate system) of the endoscope 14 can be understood based on the position and the orientation of the robot arm 12a. In the present specification, a fixed position/orientation relationship between the endoscope 14 and the robot arm 12a is referred to as "arm-camera position/orientation relationship". The arm-camera position/orientation relationship can be represented by, for example, a vector in the robot coordinate system. The coordinate system correspondence specifying unit 72 specifies a correspondence between the camera coordinate system and the robot coordinate system according to the arm-camera position/orientation relationship.

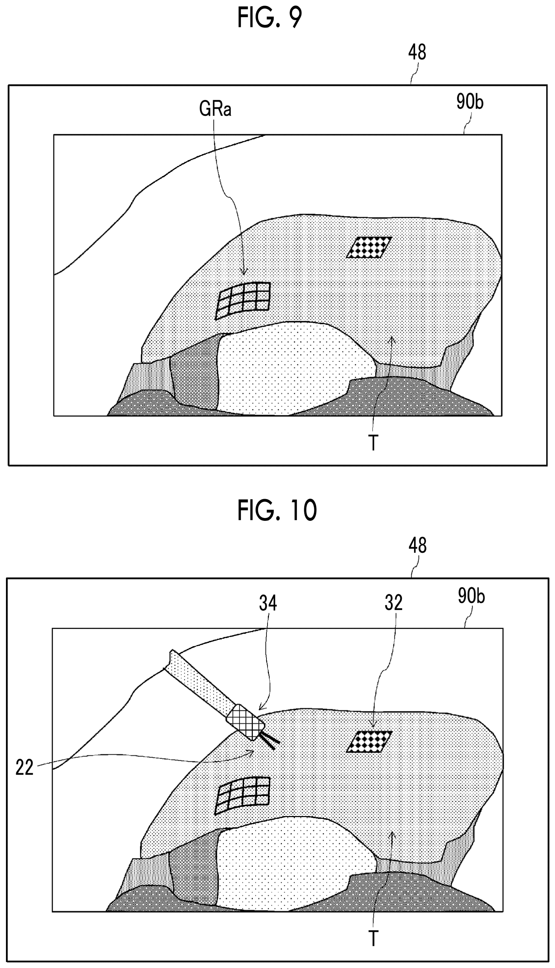

As described above, the endoscope 14 images the target tissue T to form a captured image for display 90b and transmits the captured image for display 90b to the ultrasound diagnostic apparatus 18 in a case in which the target tissue T is treated. FIG. 9 is a view showing a display example of the captured image for display 90b. The display controller 46 displays the captured image for display 90b on the display 48.

Here, the display controller 46 specifies a surface position of the treatment target portion in the three-dimensional model 56 defined in the robot coordinate system. The surface position of the treatment target portion is a position, on the surface of the target tissue T (in the present embodiment, the liver), close to the treatment target portion (in the present embodiment, the tumor) (for example, a position at a shortest distance from the treatment target portion) inside the target tissue T. The specified surface position may be represented by the point. Then, the display controller 46 displays the target position indicator indicating the surface position of the treatment target portion specified in the robot coordinate system in a superimposed manner on the display image for display 90b based on the correspondence, which is specified by the coordinate system correspondence specifying unit 72, between the camera coordinate system and the robot coordinate system.

The display controller 46 displays, as the target position indicator, a frame having a certain area including the surface position of the treatment target portion. In particular, in the present embodiment, a target position grid GRa including the scale gradations based on the dimensions in the real space (for example, representing the dimensions in the real space) in the vicinity of the surface position of the treatment target portion is displayed. The details of a process of displaying the target position grid GRa are as follows. First, the display controller 46 disposes the grid along a region having a certain area including the surface position of the treatment target portion in the three-dimensional model 56. In the present embodiment, the grid is disposed with the surface position of the treatment target portion as a center. The grid may have a lattice shape having scale gradations in a two-dimensional direction in plan view. The scale gradations are based on a predetermined dimension (for example, ∘ mm or the like) in the real space based on the scale gradations of the robot coordinate system and the scale gradations of the real space. Then, the display controller 46 forms an image in a case in which the disposed grid is viewed from the position of the endoscope 14, based on the correspondence between the camera coordinate system and the robot coordinate system, and displays the image of the grid (that is, the target position grid GRa) with the surface position of the treatment target portion in the captured image for display 90b as a reference.

In a case in which the treatment target portion is located inside the target tissue T, the operator first needs to perform the treatment (for example, incision) on the surface of the target tissue T to make the treatment target portion treatable. However, in a case in which the treatment target portion is located inside the target tissue T, the treatment target portion cannot be visually recognized from the surface of the target tissue T, so that the operator cannot determine which part of the surface of the target tissue T should be treated, by simply looking at the target tissue T. Therefore, in the related art, the operator has determined the position on the surface of the target tissue T that should be treated, based on the ultrasound tomographic image formed by transmitting and receiving the ultrasound to and from the target tissue T. However, experience and technique may be required to determine the treatment position on the surface of the target tissue T based on the ultrasound tomographic image, and it takes time and effort. In the present embodiment, as described above, by displaying the target position indicator in a superimposed manner on the captured image for display 90b, the operator can easily understand the surface position of the target tissue T that should be treated first in order to perform the treatment on the treatment target portion. In particular, by setting the target position indicator as the target position grid GRa, the operator can also understand the dimension on the surface of the target tissue T in the vicinity of the treatment target portion.

The treatment tool position/orientation detection unit 74 specifies the position and the orientation of the treatment tool 22 in the robot coordinate system based on the captured image for display 90b formed by the endoscope 14 in a case in which the target tissue T is treated.

FIG. 10 is a view showing another example of the captured image for display 90b. As described above, the captured image for display 90b is formed by imaging the target tissue T during the treatment on the target tissue T, but, in a case in which the treatment tool 22 enters an angle of view of the endoscope 14, the image of the treatment tool 22 is included in the captured image for display 90b. In particular, the captured image for display 90b may include the image of the treatment tool detection mark 34 indicating the position and the orientation of the treatment tool 22. The treatment tool position/orientation detection unit 74 analyzes the image of the treatment tool detection mark 34 in the captured image for display 90b to detect the position and the orientation of the treatment tool 22 in the camera coordinate system of the endoscope 14. Since a known method can be used as a method of detecting the position and the orientation of the treatment tool 22 in the camera coordinate system from the image of the treatment tool detection mark 34 included in the captured image for display 90b, a detailed description thereof will be omitted here.

Next, the treatment tool position/orientation detection unit 74 converts position/orientation information (hereinafter, simply referred to as "treatment tool position/orientation information") indicating the position and the orientation of the treatment tool 22 in the camera coordinate system into position/orientation information in the robot coordinate system.

Since the treatment tool 22 is held by the robot arm 12a, a position/orientation relationship between the treatment tool 22 and the robot arm 12a is fixed (not changed). In the present specification, a fixed position/orientation relationship between the treatment tool 22 and the robot arm 12a is referred to as "arm-treatment tool position/orientation relationship". The arm-treatment tool position/orientation relationship can be represented by, for example, a vector in the robot coordinate system. The treatment tool position/orientation detection unit 74 converts the treatment tool position/orientation information in the camera coordinate system into the position/orientation information in the robot coordinate system in accordance with the arm-treatment tool position/orientation relationship.

Specifically, the treatment tool position/orientation detection unit 74 acquires the position/orientation information of the robot arm 12a that holds the treatment tool 22 in the robot coordinate system and the information indicating the arm-treatment tool position/orientation relationship from the robot control device 12. The information indicating the arm-treatment tool position/orientation relationship may be input to the robot control device 12 by, for example, the operator who causes the robot arm 12a to hold the treatment tool 22. The treatment tool position/orientation detection unit 74 can obtain the treatment tool position/orientation information in the robot coordinate system by the position/orientation information of the robot arm 12a in the robot coordinate system and the arm-treatment tool position/orientation relationship. Here, by aligning the respective axial directions of the camera coordinate system and the robot coordinate system, a transformation vector from the camera coordinate system to the robot coordinate system can be obtained based on the position/orientation information of the treatment tool 22 in the camera coordinate system and the robot coordinate system. The treatment tool position/orientation detection unit 74 converts the treatment tool position/orientation information in the camera coordinate system into the position/orientation information in the robot coordinate system by using the transformation vector.

The treatment tool position/orientation detection unit 74 can also obtain the treatment tool position/orientation information in the robot coordinate system by simply using the position/orientation information of the robot arm 12a in the robot coordinate system and the arm-treatment tool position/orientation relationship without analyzing the image of the treatment tool detection mark 34 in the captured image for display 90b. However, the position and the orientation of the treatment tool 22 can be detected with higher accuracy in a case in which the position and the orientation are detected by using the treatment tool detection mark 34.

The treatment tool position/orientation detection unit 74 may further detect the position and the orientation of the target tissue T during the treatment on the target tissue T.

As described above, the captured image for display 90b also includes the image of the target detection mark 32 indicating the position and the orientation of the target tissue T during the treatment on the target tissue T. The treatment tool position/orientation detection unit 74 analyzes the image of the target detection mark 32 in the captured image for display 90b to detect the position and the orientation of the target tissue T during the treatment on the target tissue T in the camera coordinate system of the endoscope 14. Further, the treatment tool position/orientation detection unit 74 may detect the position and the orientation of the treatment tool 22 relative to the position and the orientation of the target tissue T (surface). As a result, the position and the orientation of the treatment tool 22 relative to the target tissue T, which absorb any variation in the position or the orientation of the target tissue T, can be obtained.

Since the position and the orientation of the treatment tool 22 change over time, the treatment tool position/orientation detection unit 74 continuously (in other words, in real time) detects the position and the orientation of the treatment tool 22. That is, the treatment tool position/orientation detection unit 74 continues to detect the current position and orientation of the treatment tool 22.

The current position specifying unit 76 specifies a current position, which is a position on the surface of the target tissue T in the vicinity of the distal end of the treatment tool 22 in the current position and orientation in the three-dimensional model 56 defined in the robot coordinate system, based on the position and the orientation of the treatment tool 22 in the robot coordinate system detected by the treatment tool position/orientation detection unit 74.

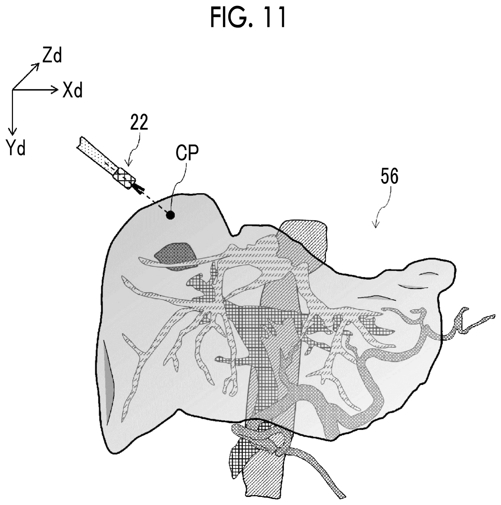

FIG. 11 is a conceptual diagram showing an aspect of a process of specifying a current position CP in the three-dimensional model 56. As described above, the position and the orientation of the treatment tool 22 in the robot coordinate system are specified by the treatment tool position/orientation detection unit 74. In FIG. 11, the specified position and orientation of the treatment tool 22 are shown. Since both the position and the orientation of the treatment tool 22 and the three-dimensional model 56 are defined in the robot coordinate system, the position/orientation relationship therebetween can be understood in the robot coordinate system. In particular, in the three-dimensional model 56, the surface position of the target tissue T is known, and thus the current position CP, which is the position on the surface of the target tissue T in the vicinity of the distal end of the treatment tool 22, can be specified. In the present embodiment, the current position specifying unit 76 specifies an intersection between a straight line (broken line in FIG. 11) extending in an extension direction of the treatment tool 22 (or the blade thereof) and the surface of the target tissue T as the current position CP.

The display controller 46 displays a current position indicator indicating the current position CP specified by the current position specifying unit 76 in the three-dimensional model 56 (that is, in the robot coordinate system) in a superimposed manner on the captured image for display 90b, based on the correspondence, which is specified by the coordinate system correspondence specifying unit 72, between the camera coordinate system and the robot coordinate system.

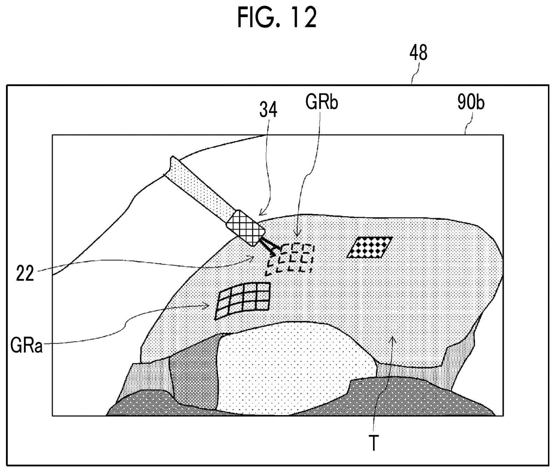

FIG. 12 is a view showing a display example of the current position indicator. A method of displaying the current position indicator is generally the same as a method of displaying the above-described target position indicator. The display controller 46 displays a frame having a certain area including the current position CP as the current position indicator. In particular, in the present embodiment, a current position grid GRb including the scale gradations based on the dimensions in the real space in the vicinity of the current position CP is displayed. The details of a process of displaying the current position grid GRb are as follows. First, the display controller 46 disposes the grid along a region having a certain area including the current position CP in the three-dimensional model 56. In the present embodiment, the grid is disposed with the current position CP as a center. The grid may have a lattice shape having scale gradations in a two-dimensional direction in plan view. The scale gradations are based on a predetermined dimension (for example, ∘ mm or the like) in the real space based on the scale gradations of the robot coordinate system and the scale gradations of the real space. Then, the display controller 46 forms an image in a case in which the disposed grid is viewed from the position of the endoscope 14, based on the correspondence between the camera coordinate system and the robot coordinate system, and displays the image of the grid (that is, the current position grid GRb) with the current position CP in the captured image for display 90b as a reference.

By displaying the current position indicator, the operator can easily understand which part of the surface of the target tissue T is to be treated at the current position of the treatment tool 22. In particular, by setting the current position indicator as the current position grid GRb, the operator can also understand the dimension of the target tissue T on the surface in the vicinity of the treatment tool 22.

As described above, the position and the orientation of the treatment tool 22 change over time, so that the current position CP also changes over time. Therefore, the position of the current position indicator (in the present embodiment, the current position grid GRb) on the captured image for display 90b also changes over time.

A rate-of-match calculation unit 78 calculates a rate of match between the surface position of the treatment target portion specified in the three-dimensional model 56 and the current position CP also specified in the three-dimensional model 56. It is possible to calculate the rate of match based on, for example, a distance between the surface position of the treatment target portion in the robot coordinate system and the current position CP. Specifically, the rate-of-match calculation unit 78 performs the calculation such that the rate of match becomes smaller as the distance between the surface position of the treatment target portion and the current position CP becomes larger, and performs the calculation such that the rate of match becomes larger as the distance between the surface position of the treatment target portion and the current position CP becomes smaller.

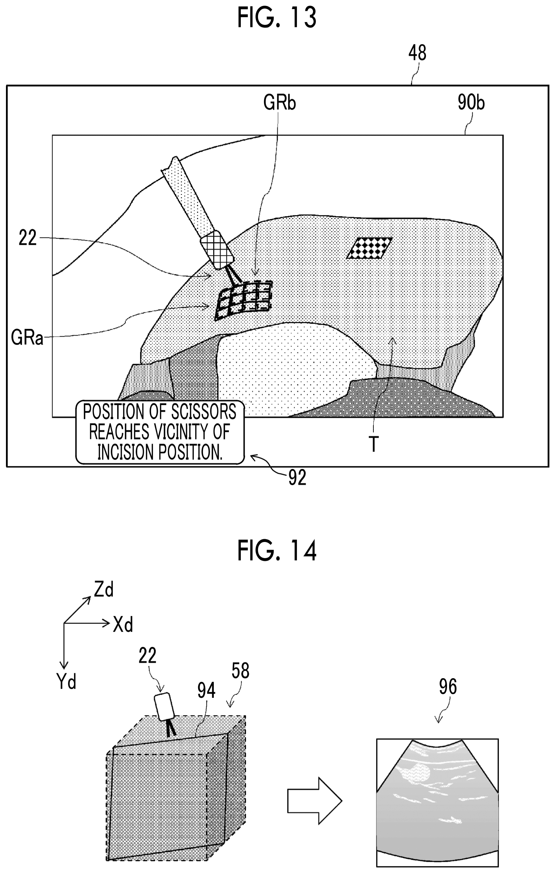

In a case in which the rate of match calculated by the rate-of-match calculation unit 78 is equal to or greater than a predetermined rate-of-match threshold value, the operator may be notified. FIG. 13 is a view showing an example of the notification that the rate of match between the surface position of the treatment target portion and the current position CP is equal to or greater than the rate-of-match threshold value. In the present embodiment, in a case in which the rate of match between the surface position of the treatment target portion and the current position CP (this can also be said to be a rate of match between the target position grid GRa and the current position grid GRb on the captured image for display 90b) is equal to or greater than the rate-of-match threshold value, the display controller 46 as a notification controller displays a message 92 as the notification on the display 48. The notification method is not limited to the display on the display 48 (that is, the notification controller is not limited to the display controller 46), and may be, for example, a method using sound or light.

With the notification, the operator can easily understand that the treatment tool 22 is present in the vicinity of the surface position of the target tissue T that should be treated first in order to perform the treatment on the treatment target portion.

A reconstruction processing unit 80 forms a reconstructed ultrasound image from the ultrasound volume data 58 defined in the robot coordinate system based on the position and the orientation of the treatment tool 22 in the robot coordinate system detected by the treatment tool position/orientation detection unit 74.

FIG. 14 is a conceptual diagram showing a concept of a process of reconstructing the ultrasound volume data 58. The reconstruction processing unit 80 can form a two-dimensional reconstructed image 96 as the reconstructed ultrasound image from the ultrasound volume data 58. In this case, the reconstruction processing unit 80 specifies a reconstructed cross-section 94 in the ultrasound volume data 58 based on the position and the orientation of the treatment tool 22 in the robot coordinate system. For example, a plane at the distal end position (cutting edge position) of the treatment tool 22 and parallel to a predetermined direction (for example, an opening direction of scissors) of the treatment tool 22 is specified as the reconstructed cross-section 94. Then, the reconstruction processing unit 80 forms, as the reconstructed ultrasound image, the two-dimensional reconstructed image 96 representing the specified reconstructed cross-section 94 by reconstructing the ultrasound volume data 58. Since a known method can be used as a method of forming the two-dimensional reconstructed image 96 by the reconstruction process, a detailed description thereof will be omitted here.

Further, the reconstruction processing unit 80 may form a three-dimensional ultrasound image as the reconstructed ultrasound image from the ultrasound volume data 58. In this case, the reconstruction processing unit 80 cuts out a part of the ultrasound volume data 58 based on the position and the orientation of the treatment tool 22 in the robot coordinate system, and performs a rendering process on the part of the ultrasound volume data 58 to form the three-dimensional ultrasound image.

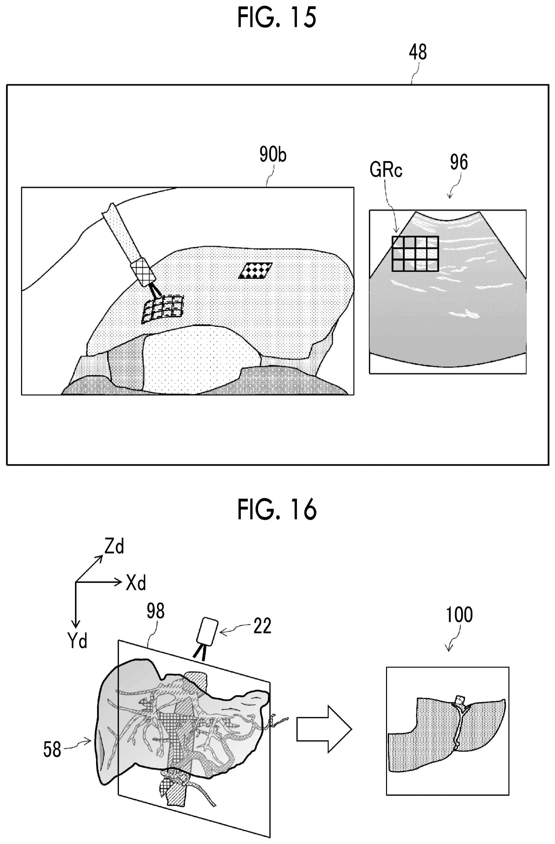

The display controller 46 displays the two-dimensional reconstructed image 96 formed by the reconstruction processing unit 80 on the display 48. FIG. 15 is a view showing a display example of the two-dimensional reconstructed image 96. As shown in FIG. 15, the display controller 46 may display the captured image for display 90b and the two-dimensional reconstructed image 96 side by side. In the example of FIG. 15, although the two-dimensional reconstructed image 96 is displayed as the reconstructed ultrasound image, the three-dimensional ultrasound image may be displayed instead of or in addition to the two-dimensional reconstructed image 96. In addition, in the example of FIG. 15, the reconstructed ultrasound image is displayed side by side with the captured image for display 90b, but the display controller 46 may display the reconstructed ultrasound image in a superimposed manner on the captured image for display 90b. In particular, the display controller 46 may display the reconstructed ultrasound image at the distal end position of the treatment tool 22 in the captured image for display 90b.

Since the position and the orientation of the treatment tool 22 change from moment to moment, the reconstruction processing unit 80 may dynamically form the reconstructed ultrasound image in accordance with the change in the position and the orientation of the treatment tool 22. In addition, in a case in which the reconstructed ultrasound image is displayed at the distal end position of the treatment tool 22, the display controller 46 may dynamically change the position at which the reconstructed ultrasound image is to be displayed, following the change in the distal end position of the treatment tool 22.

The display controller 46 may display the two-dimensional reconstructed image 96 on the display unit, and display a reconstructed image interior target position indicator representing the treatment target portion in a superimposed manner on the two-dimensional reconstructed image 96. In the present embodiment, the display controller 46 displays a reconstructed image interior target position grid GRc including the scale gradations based on the dimensions in the real space, as the reconstructed image interior target position indicator. The details of a process of displaying the reconstructed image interior target position grid GRc are as follows. First, since the treatment target portion in the ultrasound volume data 58 is specified by the process of the treatment target portion specifying unit 70, the display controller 46 can specify the region occupied by the treatment target portion in the reconstructed cross-section 94, that is, the coordinate region indicated by the treatment target portion in the two-dimensional reconstructed image 96. Then, the display controller 46 displays the reconstructed image interior target position grid GRc in the specified coordinate region. The reconstructed image interior target position grid GRc may have a lattice shape having scale gradations in a two-dimensional direction. The scale gradations may represent, for example, a predetermined dimension (for example, ∘ mm) in the real space.

By displaying the reconstructed ultrasound image, the operator can easily understand the internal structure of the target tissue T in the vicinity of the current treatment tool 22. In addition, by displaying the reconstructed image interior target position grid GRc, the operator can easily understand the treatment target region in the two-dimensional reconstructed image 96, and can also understand the dimensions of the treatment target region.

The model cross-section image formation unit 82 forms a model cross-section image representing the model cut cross-section of the three-dimensional model 56, which is specified based on the position and the orientation of the treatment tool 22 in the robot coordinate system detected by the treatment tool position/orientation detection unit 74.

FIG. 16 is a conceptual diagram showing a concept of a process of forming a model cross-section image 100. The model cross-section image formation unit 82 specifies a model cut cross-section 98 in the three-dimensional model 56 based on the position and the orientation of the treatment tool 22 in the robot coordinate system. For example, a plane at the distal end position (cutting edge position) of the treatment tool 22 and parallel to a predetermined direction (for example, an opening direction of scissors) of the treatment tool 22 is specified as the model cut cross-section 98. Then, the model cross-section image formation unit 82 forms the model cross-section image 100 representing the model cut cross-section 98 of the three-dimensional model 56.

As described above, the three-dimensional model 56 has information indicating the region occupied by the treatment target portion in the target tissue T (in the present embodiment, the liver). Therefore, in the present embodiment, the model cross-section image formation unit 82 can form the model cross-section image 100 representing the model cut cross-section 98 of at least one of the three-dimensional models 56 before the treatment target portion is removed or after the treatment target portion is removed, based on the information. The model cross-section image 100 is an example of an image from which the treatment target portion has been removed.

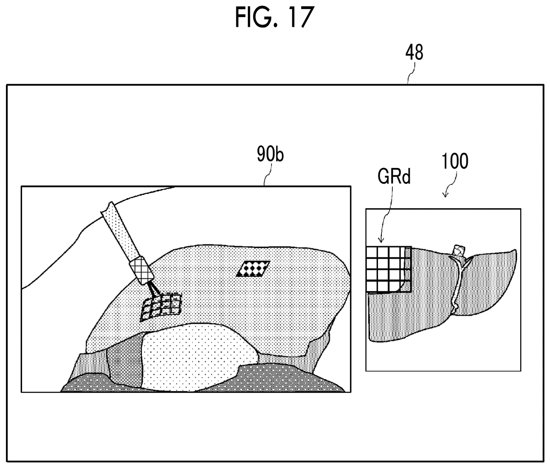

The display controller 46 displays the model cross-section image 100 formed by the model cross-section image formation unit 82 on the display 48. FIG. 17 is a view showing a display example of the model cross-section image 100. As shown in FIG. 17, the display controller 46 displays the captured image for display 90b and the model cross-section image 100 side by side.

Since the position and the orientation of the treatment tool 22 change from moment to moment, the model cross-section image formation unit 82 may dynamically form the model cross-section image 100 in accordance with the change in the position and the orientation of the treatment tool 22.

The display controller 46 may display the model cross-section image 100 on the display unit and display the model image interior target position indicator representing the treatment target portion in a superimposed manner on the model cross-section image 100. In the present embodiment, the display controller 46 displays a model image interior target position indicator GRd including the scale gradations based on the dimension in the real space, as the target position indicator in the model image. The details of a process of displaying the model image interior target position indicator GRd are as follows. First, since the treatment target portion is known in the three-dimensional model 56, the display controller 46 can specify the region occupied by the treatment target portion in the model cut cross-section 98, that is, the coordinate region indicated by the treatment target portion in the model cross-section image 100. Then, the display controller 46 displays the model image interior target position indicator GRd in the specified coordinate region. The model image interior target position indicator GRd may have a lattice shape having scale gradations in a two-dimensional direction. The scale gradations may represent, for example, a predetermined dimension (for example, ∘ mm) in the real space.

By displaying the model cross-section image 100, the operator can easily understand the internal structure of the target tissue T in the vicinity of the current treatment tool 22. In addition, by displaying the model cross-section image 100 from which the treatment target portion is removed, the operator can check a desired appearance of the target tissue T after the treatment of removing the treatment target portion. Further, by displaying the model cross-section image 100, the operator can easily understand the treatment target region in the model cross-section image 100, and can also understand the dimensions of the treatment target region.

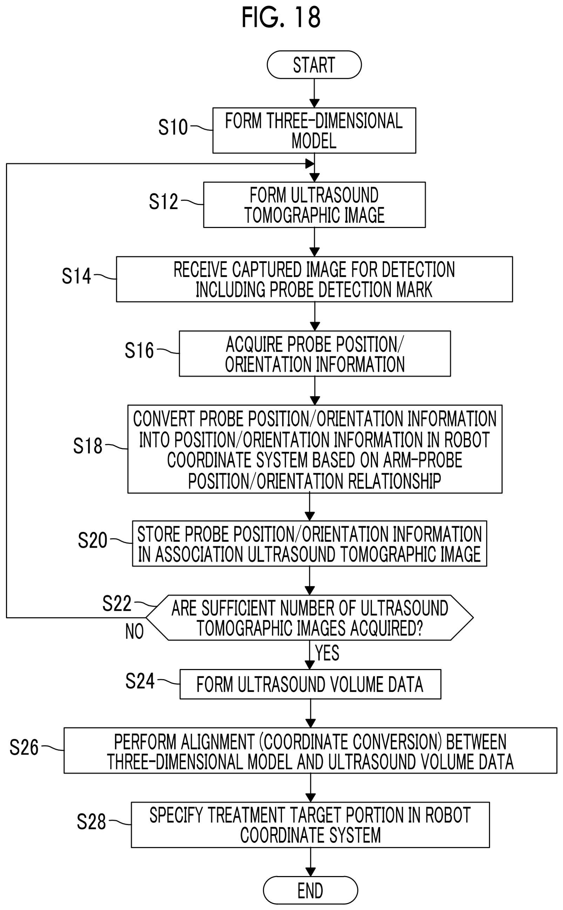

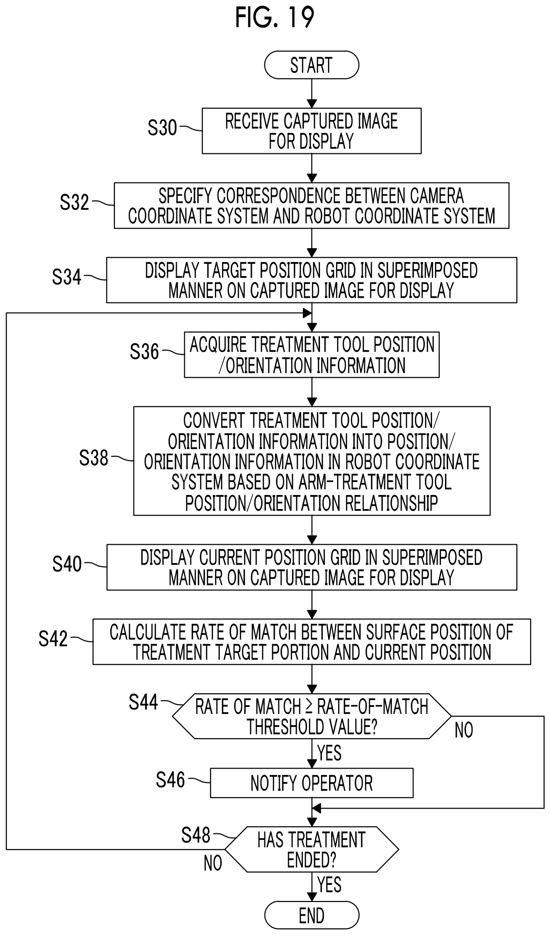

The schematic configuration of the ultrasound diagnostic apparatus 18 as the treatment support apparatus according to the present embodiment is as described above. Hereinafter, a flow of a process of the ultrasound diagnostic apparatus 18 according to the present embodiment will be described with reference to flowcharts shown in FIGS. 18 and 19.

FIG. 18 is a flowchart showing a flow of a process of specifying the treatment target portion in the ultrasound volume data 58.

In step S10, the three-dimensional model formation unit 62 forms the three-dimensional model 56 of the target tissue T based on the medical volume data received from the medical apparatus 16.

In step S12, the ultrasound probe 18a transmits and receives the ultrasound to and from the target tissue T. The image formation unit 44 forms the ultrasound tomographic image 58a for forming the ultrasound volume data 58 based on the reception signal from the ultrasound probe 18a.

In step S14, the endoscope 14 images the probe detection mark 30 during the ultrasound transmission and reception to form the captured image for detection 90a. The ultrasound diagnostic apparatus 18 receives the captured image for detection 90a from the endoscope 14.

In step S16, the probe position/orientation detection unit 64 acquires the probe position/orientation information based on the captured image for detection 90a received in step S14.

In step S18, the probe position/orientation detection unit 64 converts the probe position/orientation information in the camera coordinate system acquired in step S16 into the probe position/orientation information in the robot coordinate system based on the arm-probe position/orientation relationship.

In step S20, the probe position/orientation detection unit 64 stores the probe position/orientation information of the robot coordinate system acquired in step S16 in the memory 54 in association with the ultrasound tomographic image 58a formed in step S12.

In step S22, the volume data formation unit 66 determines whether or not a sufficient number of ultrasound tomographic images 58a for forming the ultrasound volume data 58 are acquired. In a case in which a sufficient number of ultrasound tomographic images 58a are not acquired, the process returns to step S12, and the processes of steps S12 to S20 are repeated. In a case in which a sufficient number of ultrasound tomographic images 58a are acquired, the process proceeds to step S24.

In step S24, the volume data formation unit 66 forms the ultrasound volume data 58 based on the plurality of acquired ultrasound tomographic images 58a, and stores the ultrasound volume data 58 in the memory 54. The ultrasound volume data 58 is defined in the robot coordinate system.

In step S26, the coordinate system conversion unit 68 defines the three-dimensional model 56 in the robot coordinate system by performing alignment between the three-dimensional model 56 and the ultrasound volume data 58 based on the plurality of feature points included in the three-dimensional model 56 and the plurality of feature points included in the ultrasound volume data 58.

In step S28, the treatment target portion specifying unit 70 specifies the region of the treatment target portion in the ultrasound volume data 58 based on the treatment target portion included in the three-dimensional model 56 defined in the robot coordinate system.

FIG. 19 is a flowchart showing a flow of a process of displaying the target position grid GRa and the current position grid GRb on the captured image for display 90b.

In step S30, the endoscope 14 images the target tissue T during the treatment on the target tissue T to form the captured image for display 90b. The ultrasound diagnostic apparatus 18 receives the captured image for display 90b from the endoscope 14.

In step S32, the coordinate system correspondence specifying unit 72 specifies the correspondence between the camera coordinate system of the endoscope 14 and the robot coordinate system based on the arm-camera position/orientation relationship.

In step S34, the display controller 46 specifies the surface position of the treatment target portion in the three-dimensional model 56 defined in the robot coordinate system. Then, the display controller 46 displays the target position grid GRa indicating the surface position of the treatment target portion in a superimposed manner on the captured image for display 90b based on the correspondence, which is specified in step S32, between the camera coordinate system and the robot coordinate system.

In step S36, the treatment tool position/orientation detection unit 74 specifies the position and the orientation of the treatment tool 22 in the camera coordinate system based on the captured image for display 90b including the treatment tool detection mark 34.

In step S38, the treatment tool position/orientation detection unit 74 converts the treatment tool position/orientation information in the camera coordinate system, which is acquired in step S36, into treatment tool position/orientation information in the robot coordinate system based on the arm-treatment tool position/orientation relationship.

In step S40, the current position specifying unit 76 specifies the current position CP, which is the position on the surface of the target tissue T in the vicinity of the distal end of the treatment tool 22 in the current position and orientation in the three-dimensional model 56, based on the position and the orientation of the treatment tool 22 in the robot coordinate system specified in step S38. Then, the display controller 46 displays the current position grid GRb indicating the current position CP in a superimposed manner on the captured image for display 90b based on the correspondence, which is specified in step S32, between the camera coordinate system and the robot coordinate system.

In step S42, the rate-of-match calculation unit 78 calculates the rate of match between the surface position of the treatment target portion specified in the three-dimensional model 56 and the current position CP also specified in the three-dimensional model 56.

In step S44, the display controller 46 determines whether or not the rate of match calculated in step S42 is equal to or greater than the predetermined rate-of-match threshold value. The process proceeds to step S46 in a case in which the rate of match is equal to or greater than the rate-of-match threshold value, and the process proceeds to step S48 by bypassing step S46 in a case in which the rate of match is less than the rate-of-match threshold value.

In step S46, the display controller 46 notifies the operator that the rate of match is equal to or greater than the rate-of-match threshold value.

In step S48, the controller 60 determines whether or not the treatment on the treatment target portion has ended. For example, in a case in which the operator inputs an instruction to end the treatment from the input interface 52 to the ultrasound diagnostic apparatus 18, the controller 60 determines that the treatment has ended. In a case in which the treatment has ended, the process has ended, and, in a case in which the treatment has not ended, the process returns to step S36, and the processes of steps S36 to S48 are repeated until the treatment has ended.

Although the treatment support apparatus according to the present disclosure has been described above, the treatment support apparatus according to the present disclosure is not limited to the above-described embodiment, and various modifications can be made without departing from the gist of the present disclosure.

For example, in the present embodiment, the ultrasound probe 18a is a drop-in type probe, but the ultrasound probe 18a may be other kinds of probes. The ultrasound probe 18a may be, for example, a probe that is in contact with the body surface of the subject E. Although the camera that images the probe detection mark 30, the target detection mark 32, and the treatment tool detection mark 34 is the endoscope 14, the camera need not be the endoscope 14. For example, the camera may be an extracorporeal camera that is located away from the body surface of the subject E and that images the subject E from the outside.

In addition, in each of the above-described embodiments, the controller 60 of the ultrasound diagnostic apparatus 18 has each of the functions of the three-dimensional model formation unit 62, the probe position/orientation detection unit 64, the volume data formation unit 66, the coordinate system conversion unit 68, the treatment target portion specifying unit 70, the coordinate system correspondence specifying unit 72, the treatment tool position/orientation detection unit 74, the current position specifying unit 76, the rate-of-match calculation unit 78, the reconstruction processing unit 80, and the model cross-section image formation unit 82, but each of these functions need not always be exhibited by the ultrasound diagnostic apparatus 18. These functions may be exhibited by, for example, a server computer or the like that is connected to the robot control device 12, the endoscope 14, and the ultrasound diagnostic apparatus 18 in a communicable manner. At least one of the above-described functions may be exhibited by the robot control device 12. Further, all of the above-described functions may be exhibited by cooperation of a plurality of devices without being exhibited by one device.

In addition, in each of the above-described embodiments, the grid display is superimposed and displayed on the various images, but the display need not always be the grid display. For example, instead of the grid display, a cross or circular cursor may be used to indicate the position. In addition, in each of the above-described embodiments, the scale display for understanding the actual dimension is superimposed and displayed on the various images, but these displays need not always indicate the dimension in the real space. It may also be a reference value indicating an approximate size or length.

### Explanation of References

10: treatment support system
12: robot control device
12a: robot arm
14: endoscope
16: medical apparatus
18: ultrasound diagnostic apparatus
18a: ultrasound probe
22: treatment tool
30: probe detection mark
32: target detection mark
34: treatment tool detection mark
40: transmit/receive unit
42: signal processing unit
44: image formation unit
46: display controller
48: display
50: communication interface
52: input interface
54: memory
56: three-dimensional model
58: ultrasound volume data
58a: ultrasound tomographic image
60: controller
62: three-dimensional model formation unit
64: probe position/orientation detection unit
66: volume data formation unit
68: coordinate system conversion unit
70: treatment target portion specifying unit
72: coordinate system correspondence specifying unit
74: treatment tool position/orientation detection unit
76: current position specifying unit
78: rate-of-match calculation unit
80: reconstruction processing unit
82: model cross-section image formation unit
90a: captured image for detection
90b: captured image for display
GRa: target position grid
GRb: current position grid

## Claims

1. A treatment support apparatus comprising:
a three-dimensional model acquisition unit that acquires a three-dimensional model of a target tissue, which is formed based on medical volume data acquired by a medical apparatus and is defined in a model coordinate system, the three-dimensional model having information indicating a region occupied by a treatment target portion of the target tissue;
a probe position/orientation detection unit that detects a position and an orientation of an ultrasound probe;
a volume data acquisition unit that acquires ultrasound volume data, which is formed based on reception signals obtained by transmitting and receiving ultrasound to and from the target tissue with the ultrasound probe prior to treatment on the treatment target portion, the ultrasound volume data being defined in an ultrasound data coordinate system based on the position and the orientation of the ultrasound probe in a case in which the reception signals are acquired;
a coordinate system conversion unit that defines the three-dimensional model in the ultrasound data coordinate system by performing alignment between the three-dimensional model and the ultrasound volume data based on a plurality of feature points included in the three-dimensional model and a plurality of feature points included in the ultrasound volume data; and
a treatment target portion specifying unit that specifies a region of a treatment target portion in the ultrasound volume data based on the treatment target portion included in the three-dimensional model defined in the ultrasound data coordinate system.

2. The treatment support apparatus according to claim 1,
wherein the ultrasound probe is held by a robot arm controlled by a robot control device,
the ultrasound data coordinate system corresponds to a robot coordinate system which is used by the robot control device to determine a position and an orientation of the robot arm and is further based on a fixed position/orientation relationship between the ultrasound probe and the robot arm that holds the ultrasound probe, and
the treatment support apparatus further comprises:
a coordinate system correspondence specifying unit that specifies a correspondence between a camera coordinate system, which is a coordinate system of a camera held by the robot arm controlled by the robot control device, and the robot coordinate system based on a fixed position/orientation relationship between the camera and the robot arm; and
a display controller that displays, on a display unit, a target position indicator indicating a surface position of the treatment target portion in the three-dimensional model in a superimposed manner on a captured image for display formed by imaging the target tissue with the camera based on the correspondence between the camera coordinate system and the robot coordinate system.

3. The treatment support apparatus according to either claim 1 or claim 2,
wherein the ultrasound probe is held by a robot arm controlled by a robot control device,
the ultrasound data coordinate system corresponds to a robot coordinate system which is used by the robot control device to determine a position and an orientation of the robot arm and is further based on a fixed position/orientation relationship between the ultrasound probe and the robot arm that holds the ultrasound probe, and
the treatment support apparatus further comprises:
a coordinate system correspondence specifying unit that specifies a correspondence between a camera coordinate system, which is a coordinate system of a camera held by the robot arm controlled by the robot control device, and the robot coordinate system based on a fixed position/orientation relationship between the camera and the robot arm;
a treatment tool position/orientation detection unit that specifies a position and an orientation, in the robot coordinate system, of a treatment tool which is used for performing the treatment on the treatment target portion and is held by the robot arm controlled by the robot control device;
a current position specifying unit that specifies a current position which is a position on a surface of the target tissue in vicinity of a distal end of the treatment tool in a current position and orientation in the three-dimensional model; and
a display controller that displays, on a display unit, a current position indicator indicating the current position in a superimposed manner on a captured image for display formed by imaging the target tissue with the camera based on a correspondence between the camera coordinate system and the ultrasound data coordinate system.

4. The treatment support apparatus according to any one of the previous claims, in particular claim 2, further comprising:
a treatment tool position/orientation detection unit that specifies a position and an orientation, in the robot coordinate system, of a treatment tool which is used for performing the treatment on the treatment target portion and is held by the robot arm controlled by the robot control device; and
a current position specifying unit that specifies a current position that is a position on a surface of the target tissue in vicinity of a distal end of the treatment tool in a current position and orientation in the three-dimensional model,
wherein the display controller further displays a current position indicator indicating the current position in a superimposed manner on the captured image for display based on a correspondence between the camera coordinate system and the robot coordinate system.

5. The treatment support apparatus according to any one of the previous claims, in particular claim 4, further comprising:
a rate-of-match calculation unit that calculates a rate of match between the surface position of the treatment target portion and the current position of the treatment tool; and
a notification controller that notifies an operator in a case in which the rate of match is equal to or greater than a rate-of-match threshold value.

6. The treatment support apparatus according to any one of the previous claims, in particular claim 2 or 4,
wherein the target position indicator includes scale gradations based on dimensions in a real space in vicinity of the surface position of the treatment target portion.

7. The treatment support apparatus according to any one of the previous claims, in particular claim 3 or 4,
wherein the current position indicator includes scale gradations based on dimensions in a real space in vicinity of the current position.

8. The treatment support apparatus according to any one of the previous claims, in particular claim 3 or 4, further comprising:
a reconstruction processing unit that specifies a reconstructed cross-section based on a position and an orientation of the treatment tool in the robot coordinate system and reconstructs the ultrasound volume data to form a two-dimensional reconstructed image representing the reconstructed cross-section,
wherein the display controller displays the two-dimensional reconstructed image on a display unit and displays a reconstructed image interior target position indicator indicating the treatment target portion in the reconstructed cross-section in a superimposed manner on the two-dimensional reconstructed image.

9. The treatment support apparatus according to any one of the previous claims, in particular claim 3 or 4, further comprising:
a model cross-section image formation unit that specifies a model cut cross-section based on a position and an orientation of the treatment tool in the robot coordinate system and forms a model cross-section image representing the model cut cross-section of at least one of the three-dimensional models before the treatment target portion is removed or after the treatment target portion is removed,
wherein the display controller displays the model cross-section image on a display unit.

10. The treatment support apparatus according to any one of the previous claims, in particular claim 9,
wherein the display controller displays the model cross-section image on the display unit and displays a model cross-section image interior target position indicator indicating the treatment target portion in a superimposed manner on the model cross-section image.

11. A treatment support program causing a computer to function as:
a three-dimensional model acquisition unit that acquires a three-dimensional model of a target tissue, which is formed based on medical volume data acquired by a medical apparatus and is defined in a model coordinate system, the three-dimensional model having information indicating a region occupied by a treatment target portion of the target tissue;
a probe position/orientation detection unit that detects a position and an orientation of an ultrasound probe;
a volume data acquisition unit that acquires ultrasound volume data, which is formed based on reception signals obtained by transmitting and receiving ultrasound to and from the target tissue with the ultrasound probe prior to treatment on the treatment target portion, the ultrasound volume data being defined in an ultrasound data coordinate system based on the position and the orientation of the ultrasound probe in a case in which the reception signals are acquired;
a coordinate system conversion unit that defines the three-dimensional model in the ultrasound data coordinate system by performing alignment between the three-dimensional model and the ultrasound volume data based on a plurality of feature points included in the three-dimensional model and a plurality of feature points included in the ultrasound volume data; and
a treatment target portion specifying unit that specifies a region of a treatment target portion in the ultrasound volume data based on the treatment target portion included in the three-dimensional model defined in the ultrasound data coordinate system.
